# EUROPÄISCHE PATENTSCHRIFT

(11) **EP 1 958 581 B1**
(45) Veröffentlichungstag und Bekanntmachung des Hinweises auf die Patenterteilung: **21.09.2011**
(21) Anmeldenummer: 08002587.7
(22) Anmeldetag: 13.02.2008
(51) Int. Cl.: A61B 17/82, A61B 17/00

(54) **Implantat zur Fixierung von Knochenteilen insbesondere des Sternums**
Implant for securing bone parts in particular of the sternum
Implant de fixation de parties d'os particulèrement du sternum

(30) Priorität: 13.02.2007 DE 102007008184
(43) Veröffentlichungstag der Anmeldung: 20.08.2008
(73) Patentinhaber: Aesculap AG, 78532 Tuttlingen (DE)
(72) Erfinder: Morales, Pedro, 78532 Tuttlingen/Donau (DE); Weißhaupt, Dieter, 78194 Immendingen (DE); Lutze, Theodor, 78582 Balgheim (DE); Dworschak, Manfred, 78589 Dürbheim (DE)
(74) Vertreter: Patentanwälte Ruff, Wilhelm, Beier, Dauster & Partner

(56) Entgegenhaltungen:
- EP-A- 0 238 219
- WO-A-02/24088
- DE-B4- 10 326 690
- DE-U1-202006 015 031
- DE-U1-202006 015 037
- US-A1- 2005 240 189
- US-A1- 2007 299 520

## Beschreibung

Die Erfindung betrifft ein Implantat, insbesondere zur gegenseitigen Fixierung von zwei miteinander zu verbindenden Knochenteilen des Sternums, mit einem ersten Anlageelement zur Anlage an der Innenseite von Knochenteilen, mindestens einem an dem ersten Anlageelement festgelegten, quer von diesem abstehenden Spannelement und einem zweiten Anlageelement zur Anlage an der Außenseite von Knochteilen, welches mittels des mindestens einen durch einen Zwischenraum zwischen den Knochenteilen hindurchgeführten Spannelements gegen das erste Anlageelement spannbar ist.

Zu den am häufigsten zu verbindenden Knochenteilen zählen Knochenteile des Brustbeins (Sternums). Eine Durchtrennung des Sternums kommt regelmäßig bei einer Herzoperation, beispielsweise Bypass-Operationen, in Betracht. Ein entsprechendes Implantat zur Festlegung von zwei miteinander zu verbindenden Sternumteilen ist beispielsweise aus der DE 103 26 690 B4 bekannt. Durch das Implantat werden die zu verbindenden Sternumteile in einer angenäherten Position gehalten, so dass eine knöcherne Verwachsung der angenäherten Sternumteile ermöglicht wird.

In einigen Fällen wird jedoch im Rahmen einer Reoperation eine erneute Durchtrennung der Knochenteile und damit eine Entfernung des eingesetzten Implantates erforderlich. So werden zum Beispiel 6 % bis 7 % der am Herzen operierten Patienten nach einigen Monaten, zum Teil auch erst nach einigen Jahren, reoperiert. Dazu muss gewöhnlich der Brustkorb wieder geöffnet werden, wobei das Sternum längs einer Trennlinie durchtrennt wird.

Als nachteilig erweist sich vor allem, dass die Oberflächen der eingesetzten Implantate häufig mit natürlichem Knochen- und Bindegewebe überwachsen sind. Dies ist um so stärker der Fall, je länger die Implantation zurückliegt. Dies kann zu einer sehr festen Verbindung des Implantats mit den ursprünglich zu verbindenden Knochenteilen führen, wodurch die im Rahmen einer Reoperation erforderliche Entfernung des Implantats wesentlich erschwert werden kann.

Die DE 20 2006 015 031 U1 offenbart ein Sternumverschluß zur Festlegung von zwei miteinander zu verbindenden Sternumteilen mit einem inneren und äußeren Anlageelement, wobei die Anlageelemente zur Verhinderung einer Knochenmaterialanhaftung eine Beschichtung aus Polytetrafluorethylen aufweisen können. Diese Druckschrift offenbart die Merkmale des Oberbegriffs von Auspruch 1.

Aus der WO 02/24088 A2 ist ein medizintechnisches Produkt zur Behandlung von distalen Radiusfrakturen bekannt, welches eine antimikrobielle Beschichtung auf Basis eines Silberzeolithen aufweisen kann.

Aus der EP 0 238 219 A1 geht ein Sternumverschluß hervor, welches sich nach Art eines Kabelbinders einsetzen lässt und aus einem bioverträglichem Metall hergestellt sein kann, welches mit einem bioverträglichen Polymer wie beispielsweise einem Polyolefin beschichtet sein kann.

Die DE 20 2006 015 037 U1 offenbart ein Sternumverschluß mit einer Abdeckkappe aus Polyetheretherketon.

Es ist daher Aufgabe der Erfindung ein Implantat bereitzustellen, das ein möglichst komplikationsfreies Durchtrennen von natürlich miteinander verwachsenen Knochteilen bei einer Reoperation ermöglicht.

Diese Aufgabe wird bei einem Implantat der eingangs beschriebenen Art erfindungsgemäß dadurch gelöst, dass zumindest das erste Anlageelement einteilig ausgebildet ist und das Implantat an seiner Oberfläche zumindest teilweise eine Antihaftschicht zur Verhinderung von postoperativen Gewebeadhäsionen (Gewebeverwachsungen) aufweist. Die Antihaftschicht kann insbesondere vollständig aus einem antihaftenden Material bestehen.

Unter der Innenseite eines Knochenteils im Sinne der vorliegenden Erfindung soll die dem Chirurgen abgewandte Seite des betreffenden Knochteils verstanden werden (proximale Seite). Demgegenüber soll es sich im Falle der Außenseite eines Knochenteils im Sinne der vorliegenden Erfindung um die dem Chirurgen zugewandte Seite des betreffenden Knochenteils handeln (distale Seite).

Durch die Erfindung wird ein Implantat für den Knochenverschluss bereitgestellt, welches durch eine zumindest teilweise Oberflächenschicht mit einem antihaftenden Material unerwünschte Verwachsungen mit Knochen-, Knorpel- und/oder Bindegewebe an der Implantationsstelle weitgehend verhindert oder doch wenigstens deutlich erschwert. Auf diese Weise können im Falle einer notwendigen erneuten Durchtrennung des Knochens insbesondere die proximalen, d.h. für den Chirurgen schwerer zugänglichen, Elemente des Implantates unaufwendig und für den Patienten risikoarm entfernt werden.

In einer besonders bevorzugten Ausführungsform handelt es sich bei dem erfindungsgemäßen Implantat um einen sogenannten Sternumverschluss. Mit Hilfe eines solchen Sternumverschlusses können durchtrennte Sternumteile (Knochenteile des Brustbeins) einander angenähert und in dieser angenäherten Position fixiert werden, so dass eine natürliche Verbindung der Sternumteile durch knöcherne Verwachsungsprozesse eintreten kann. In dieser Ausführungsform dient das erste Anlageelement zur Anlage an der Innenseite des Sternums und das zweite Anlageelement zur Anlage an der Außenseite des Sternums.

Gemäß einer weiteren bevorzugten Ausführungsform handelt es sich bei dem Implantat um eine Anordnung zum Fixieren eines aus der Schädelkapsel zum Zwecke eines operativen Eingriffs herausgetrennten Knochenstückes am verbliebenen Schädelbein.

Das Implantat besteht vorzugsweise aus einem bioverträglichen Metall oder einer bioverträglichen Metalllegierung. Als geeignete Metalle kommt insbesondere Titan in Betracht. Bei den Metalllegierungen kann es sich beispielsweise um Titanlegierungen, insbesondere um Titan-Aluminiüm-Legierungen, handeln. Beispielsweise kann die Titanlegierung Ti6Al4V verwendet werden. Ebenso kann das unbeschichtete Implantat aus einem speziellen Stahl bestehen.

Die Anlageelemente weisen zweckmäßigerweise eine im wesentlichen ebene Anlagefläche zur Anlage an die miteinander zu verbindenden Knochenteile auf. Bevorzugt sind die Anlageelemente rechteckig und insbesondere plattenförmig ausgebildet. Besonders bevorzugt sind die Anlageelemente tellerförmig ausgebildet. Die Anlageelemente können Tellerdurchmesser von 11 mm, 16 mm und/oder 20 mm aufweisen. Weiterhin können die Anlageelemente eine Wölbung unter Ausbildung einer konvexen und konkaven Seite besitzen. Zumindest das erste Anlageelement ist erfindungsgemäß einteilig, d.h. einstückig bzw. untrennbar, ausgebildet. Vorzugsweise ist auch das zweite Anlageelement einteilig ausgebildet. Die Anlageelemente sind durch das mindestens eine Spannelement, das durch den Zwischenraum zwischen den miteinander zu verbindenden Knochenteilen hindurchragt, gegeneinander verspannbar. Auf diese Weise wird erreicht, dass die Anlageelemente im wesentlichen parallel zu diesem Zwischenraum und parallel zueinander angeordnet sind. Das mindestens eine Spannelement kann beispielsweise stiftförmig ausgebildet sein. Mit besonderem Vorteil ist das mindestens eine Spannelement als Gewindeschraube ausgebildet, so dass im Falle einer Reoperation das mindestens eine Spannelement - nach Abnahme des zweiten Anlageelementes - aus dem ersten Anlageelement herausgeschraubt werden kann.

Das erfindungsgemäße Implantat kann grundsätzlich ein Spannelement oder mehrere Spannelemente aufweisen. Für den Fall, dass es sich bei dem Implantat um eine Anordnung zur Refixierung von craniotomierten Knochendeckeln an der Schädelkalotte handelt, kann es bevorzugt sein, wenn das Implantat lediglich ein Spannelement aufweist. Handelt es sich bei dem Implantat beispielsweise um einen Sternumverschluß, können zwei Spannelemente bevorzugt sein. Bevorzugt weist das Implantat zwei Spannelemente auf. Die Verspannung des zweiten Anlageelementes relativ zum ersten Anlageelement kann beispielsweise mit einem geeigneten Spannwerkzeug erreicht werden. Das Spannwerkzeug kann an das mindestens eine Spannelement angelegt werden, um das zweite Anlageelement um eine bestimmte Wegstrecke in Richtung auf das erste Anlageelement zu verschieben. Im Falle von zwei oder mehr Spannelementen können diese an ihrem dem ersten Anlageelement gegenüberliegenden Ende durch eine Brücke miteinander verbunden werden. Dadurch werden die Spanneelemente stabilisiert. Zudem werden die freien Enden der Spannelemente abgedeckt, so dass Gewebeschädigungen durch die freien Enden der Spannelemente vermieden werden können.

Die Antihafteigenschaften aufweisenden Oberflächen des Implantats (Antihaftschicht) sind in einer weiteren Ausführungsform von einer mindestens einlagigen Antihaftbeschichtung gebildet. In einer anderen möglichen Ausführungsform weist das Implantat neben einer Antihaftschicht eine Antihaftbeschichtung auf, welche vorzugsweise die Antihaftschicht zumindest teilweise bedeckt.

In einer bevorzugten Ausführungsform weisen die Oberflächen der Anlageelemente zumindest teilweise die Antihaftschicht auf. Beispielsweise können die Anlagenelemente nur an ihrer Ober- oder Unterseite die Antihaftschicht aufweisen, insbesondere an den jeweils dem Brustbein zugewandten Flächen. Bevorzugt weist zumindest das erste (innere) Anlageelement die Antihaftschicht auf. Erfindungsgemäß kann es auch vorgesehen sein, dass nur das erste Anlageelement zumindest teilweise, insbesondere vollständig, beschichtet ist. Dies ist deswegen vorteilhaft, da sich das erste Anlageelement nach Implantation auf der proximalen Knochenseite befindet und damit nur schwer zugänglich ist. Die in der Einleitung problematisierten Gewebeverwachsungen würden anderenfalls eine Entfernung des ersten Anlageelements und damit eine vollständige Entfernung des Implantats zusätzlich erschweren.

Grundsätzlich können die Anlageelemente des erfindungsgemäßen Implantats vollständig, einschließlich an den Anlageelementen angeordneter Haltevorsprünge, mit einem antihaftenden Material beschichtet sein. Ist auch das zweite (äußere) Anlageelement beschichtet, dann können Rastelemente frei von einer Beschichtung sein.

In einer bevorzugten Ausführungsform sind an den Anlageelementen angeordnete Haltevorsprünge frei von der Antihaftschicht. Dadurch ist ein besonders wirkungsvolles Eindringen der Haltevorsprünge in das zu verbindende Knochengewebe möglich. Die Haltevorsprünge weisen zweckmäßigerweise in Richtung auf das jeweils andere Anlageelement. Sie dienen zur Fixierung der Anlageelemente an die zu verbindenden Knochenteile, indem sie beim Andrücken der Halteelemente an die Innen- bzw. Außenseite der Knochenteile in das Knochengewebe eintreten. Dadurch werden die Anlageelemente an den Knochenteilen verkrallt.

Die Haltevorsprünge sind vorzugsweise spitz ausgebildet und können in unterschiedlichen Formen vorliegen, beispielsweise in Form von Zacken, Zapfen oder Kegeln. Weiterhin können die Halteelemente gegenüber dem mindestens einen Spannelement geneigt sein. Es ist besonders günstig, wenn die Haltevorsprünge des jeweiligen Anlageelementes aufeinander zu geneigt sind, da somit das seitliche Einschieben der Knochenteile zwischen die Anlageelemente erleichlert wird.

Die Haltevorsprünge bestehen regelmäßig aus einem Metall oder einer Metalllegierung. Ebenso können sie aus einem speziellen Stahl bestehen. Die Haltevorsprünge bestehen zweckmäßigerweise aus demselben Material wie die Anlageelemente. Die Haltevorsprünge und die Anlageelemente sind bevorzugt einstückig ausgebildet.

Die Antihaftschicht weist ein Polyetherketon auf. Bei den Polyetherketonen handelt es sich besonders bevorzugt um Polyetheretherketon (PEEK). Als weitere geeignete Polyetherketone sind Polyetheretheretherketone (PEEEK), Polyetheretherketonetherketone (PEEKEK) und Polyetherketonketone (PEKK) zu nennen. Die Verwendung von Polyetherketonen zur Beschichtung des erfindungsgemäßen Implantats ist insbesondere deswegen bevorzugt, da es sich um ein wiederholt sterilisierbares und insbesondere röntgendurchlässiges Polymer handelt.

In einer weiteren vorteilhaften Ausführungsform weist die Antihaftschicht ein resorbierbares Polymer, insbesondere einen aliphatischen Polyester, auf. Bei den Polyestern kann es sich zum Beispiel um Poly-p-Dioxanon, Trimethylencarbonat, Polyglykolid und/oder Polylactid handeln. Besonders bevorzugt handelt es sich bei den Polyestern um Polylactid und/oder Polylactidcopolymere.

Es ist bevorzugt, wenn die Antihaftschicht aus Polyetheretherketon (PEEK) oder Polyetheretherketon (PEEK) und Polylactid bzw. einem Polylactidcopolymer besteht.

In einer weiteren Ausführungsform weist die Antihaftschicht ein natürliches Polymer auf. Bei dem natürlichen Polymer handelt es sich insbesondere um ein Polysaccharid oder ein Protein. Als Polysaccharid kommt grundsätzlich auch ein modifiziertes, insbesondere oxidiertes, Polysaccharid in Frage. Bei dem Protein handelt es sich insbesondere um ein Stütz- oder Gerüstprotein. Bevorzugt weist die Antihaftschicht ein natürliches Polymer aus der Gruppe, umfassend Cellulose, Carboxymethylcellulose (CMC), Amylose, Amylopektin, Stärke, Hyaluronsäure, Dextran, Kollagen, Gelatine und/oder Elastin, auf. In einer möglichen Ausführungsform besteht die Antihaftschicht aus einem der in diesem Abschnitt genannten natürlichen Polymeren.

Es kann weiterhin bevorzugt sein, dass die Antihaftschicht mehrschichtig ist. Die Antihaftschicht kann insbesondere eine Mischung aus den in den vorangegangenen Ausführungsformen beschriebenen Polymeren aufweisen bzw. aus einer solchen Mischung bestehen. Bezüglich weiterer Einzelheiten zu den in Frage kommenden Polymeren wird daher auf die bisherige Beschreibung Bezug genommen.

Zur Beschichtung des Implantats mit einem Polymer kommen grundsätzlich alle dem Fachmann geläufigen Beschichtungstechniken in Betracht. Erfindungsgemäß wird das Polyetherketon in Pulverform durch Pulverbeschichtung aufgetragen. Eine derartige Beschichtung lässt sich insbesondere mit einem Wirbelsinterverfahren oder mit elektrostatischen Verfahren durchführen. Weiterhin kann das unbeschichtete Implantat in eine Lösung der in den vorangegangenen Ausführungsformen beschriebenen Polymere getaucht werden. Die Beschichtung des Implantats kann ebenso mittels eines geeigneten Spritzverfahrens durchgeführt werden. Im Übrigen ist auch eine Beschichtung durch Plasmabehandlung des Implantats, beispielsweise durch Plasma Vapour Deposition, möglich.

In einer weiteren möglichen Ausführungsform weist die Antihaftschicht Nanostrukturen auf.

In einer bevorzugten Ausführungsform ist die Antihaftschicht durch eine Oberflächenbehandlung des Implantats gebildet. Zur Erzeugung der Antihaftschicht kann das Implantat beispielsweise gebeizt und/oder aufgeraut werden. Weiterhin kann das Implantat einem elektrochemischen Prozess unterworfen werden, um die Antihaftschicht zu erzeugen.

Weiterhin kann es vorgesehen sein, dass die Antihaftschicht des erfindungsgemäßen Implantats aus einer Kombination der in den vorangegangenen Ausführungsformen beschriebenen Antihaftmaterialien gebildet ist. Beispielsweise kann die Antihaftschicht aus einer Metalloxidschicht bestehen, die mit Polyetherketon beschichtet ist.

In einer besonders bevorzugten Ausführungsform weist das Implantat an seiner Oberfläche neben der Antihaftschicht zumindest teilweise auch eine biologisch aktive Beschichtung auf. Unter einer biologisch aktiven Beschichtung soll eine Beschichtung verstanden werden, welche das natürliche Knochenwachstum unterstützt bzw. fördert. Die besagte Beschichtung besteht vorzugsweise aus einem Apatit. Als geeignete Apatite kommen grundsätzliche Fluor-, Chlor- und/oder Hydroxylapatite in Betracht. Bevorzugt handelt es sich bei der biologisch aktiven Beschichtung um eine Beschichtung aus Hydroxylapatit. Erfindungsgemäß kann es insbesondere vorgesehen sein, dass das eine Anlageelement eine Antihaftschicht und das andere Anlageelement eine biologisch aktive Beschichtung aufweist. Ggf. kann auch das Spannelement eine biologisch aktive Beschichtung aufweisen, um den Verwachsungsprozeß zwischen den zu verbindenden Knochenteilen zusätzlich zu beschleunigen. Für den Fall, dass es sich bei dem Implantat um eine Anordnung zur Refixierung von craniotomierten Knochendeckeln an der Schädelkalotte handelt, ist es besonders bevorzugt, wenn das erste Anlageelement eine Antihaftschicht, insbesondere aus einem Polyetherketon, und das zweite Anlageelement eine biologisch aktive Beschichtung, vorzugsweise aus einem Apatit, aufweist.

Weiterhin kann es vorgesehen sein, dass die in der vorherigen Ausführungsform beschriebene biologisch aktive Beschichtung Wachstumsfaktoren, beispielsweise BMPs (Bone Morphogenetic Proteins) aufweist.

Die Antihaftschicht und/oder eine biologisch aktive Beschichtung besitzen bevorzugt eine Dicke zwischen 0,1 µm und 0,5 mm. Weiterhin kann die durch Pulverbeschichtung erzeugte Antihaftschicht eine Dicke zwischen 0,15 mm und 0,5 mm, insbesondere zwischen 0,15 mm und 0,3 mm, besitzen.

Die vorliegende Erfindung betrifft des Weiteren auch die Verwendung eines Gewebeverklebungen verhindernden Materials und/oder eines biologisch aktiven Materials zur Beschichtung eines Implantats mit einem ersten Anlageelement zur Anlage an der Innenseite von Knochenteilen, mindestens einem an dem ersten Anlageelement festgelegten, quer von diesem abstehenden Spannelement und einem zweiten Anlageelement zur Anlage an der Außenseite von Knochenteilen, welches mittels des mindestens einen durch einen Zwischenraum zwischen den Knochenteilen hindurchgeführten Spannelements gegen das erste Anlageelement spannbar ist. Bezüglich der möglichen Materialien bzw. Materialkombinationen wird auf die bisherige Beschreibung verwiesen. Bevorzugt handelt es sich bei dem Gewebeverklebungen verhindernden Material um ein Polyetherketon und bei dem biologisch aktiven Material um ein Apatit, vorzugsweise Hydroxylapatit.

Weiterhin kann die Antihaftschicht Wirkstoffe aufweisen. Bei den Wirkstoffen handelt es sich insbesondere um antimikrobiell wirksame Substanzen, beispielsweise um Antibiotika. Bevorzugt handelt es sich bei den Substanzen um Silber oder Silbersalze.

Weitere Merkmale und Einzelheiten der Erfindung ergeben sich aus der Figurenzeichnung einer bevorzugten Ausführungsform der Erfindung sowie aus der dazugehörenden Beschreibung. Es zeigen schematisch:
- Figur 1:: eine bevorzugte Ausführungsform des erfindungsgemäßen Implantates,
- Figur 2:: eine bevorzugte Ausführungsform zum Fixieren eines aus der Schädelkapsel herausgetrennten Knochenstückes am verbliebenen Schädelbein,
- Figur 3:: eine Längsschnittansicht durch ein erfindungsgemäßes Implantat für Knochenplatten mit den wesentlichen Teilen eines Anlegeinstrumentes (strichpunktiert),
- Figur 4:: eine vergrößerte Längsschnittansicht des Bereiches A in Figur 3.

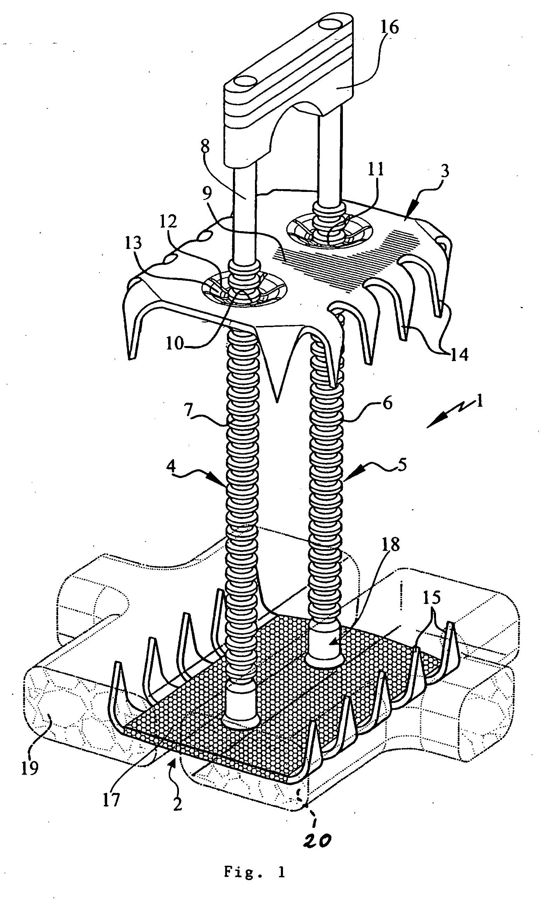

Bei dem in Figur 1 schematisch dargestellten Implantat 1 handelt es sich um einen sogenannten Sternumverschluss. Der Sternumverschluss 1 weist ein erstes Anlageelement 2 und ein zweites Anlageelement 3 auf, welche über zwei als Spannelemente wirkende Raststifte 4 und 5 miteinander verbunden sind und gegeneinander gespannt werden können. Die Raststifte 4 und 5 sind an dem plattenförmigen ersten Anlageelement 2 so angebracht, dass sie senkrecht von der Oberseite des ersten Anlageelementes 2 abstehen. Die Anlageelemente 2 und 3 sowie die Raststifte 4 und 5 bestehen aus einem Metall, beispielsweise aus Titan. Die Oberseite des ersten Anlageelementes 2 bildet eine im Wesentlichen ebene Anlagefläche aus. Die beiden Raststifte 4 und 5 tragen oberhalb des ersten Anlageelementes 2 eine größere Anzahl an als Rastelemente dienende Umfangsrippen 6. Die Raststifte 4 und 5 bilden mit den Umfangsrippen 6 einen Rastabschnitt 7 aus, an den sich zum freien Ende der Raststifte 4 und 5 hin jeweils ein Verlängerungsabschnitt 8 mit glatter Oberfläche anschließt. Auf die Verlängerungsabschnitte 8 der beiden Raststifte 4 und 5 ist das zweite Anlageelement 3 aufgesteckt. Dieses weist ein etwa rechteck- und plattenförmiges Mittelstück 9 mit einer ebenen Unterseite auf sowie zwei das Mittelstück 9 durchsetzende Öffnungen 10 und 11, durch welche die Verlängerungsabschnitte 8 der beiden Raststifte 4 und 5 hindurchragen. Von den Öffnungen 10 und 11 gehen radiale Einschnitte 12 aus, welche den Randbereich der Öffnungen 10 und 11 in voneinander getrennte Rastzungen bildende, federnde Lappen 13 unterteilen. Die Lappen 13 können elastisch aus ihrer Ausgangslage verbogen werden, wenn das zweite Anlageelement 3 in Richtung auf das erste Anlageelement 2 verschoben wird und die Lappen 13 dabei an den Umfangsrippen 6 entlang gleiten. Dadurch lässt sich das zweite Anlageelement 3 zwar in Richtung des ersten Anlageelementes 2 verschieben, nicht aber umgekehrt. An beiden Längskanten des Mittelstückes 9 ist das metallische Material des zweiten Anlageelementes 3 in Richtung auf das erste Anlageelement 2 etwa rechtwinklig abgebogen und bildet dort eine Reihe von spitzen pfeilförmigen Haltevorsprüngen 14 aus. Die Haltevorsprünge 14 sind mit dem zweiten Anlageelement 3 einstückig ausgebildet.

Auch das erste Anlageelement 2 trägt an seinen beiden Längskanten in Richtung auf das zweite Anlageelement 3 weisende spitze pfeilförmige Haltevorsprünge 15, welche ebenfalls einstückig mit dem ersten Anlageelement 2 ausgebildet sind. Die beiden Raststifte 4 und 5 sind an den Enden der Verlängerungsabschnitte 8 über eine Brücke 16 miteinander verbunden.

Das erste Anlageelement 2 ist mit Ausnahme der Haltevorsprünge 15 mit einer Antihaftschicht 17 aus Polyetheretherketon (PEEK) vollständig beschichtet. Die Antihaftschicht 17 kann beispielsweise durch eine geeignete Sprüh- oder Spritztechnik auf das erste Anlageelement 2 aufgebracht werden.

Die beiden Raststifte 4 und 5 tragen an ihrem unteren Ende jeweils einen vergrößerten Kopf 18, welcher in einer in der Figur nicht dargestellten komplementären Vertiefung des ersten Anlageelementes 2 eingreift.

Die Implantation des Sternumverschlusses 1 erfolgt durch die Anlage des ersten Anlageelementes 2 an die Innenseite bzw. Innenfläche von zwei durch einen Trennschnitt voneinander getrennten und aufgespreizten Knochenteilen 19 und 20 des Sternums (Sternumteile). Durch den infolge der Aufspreizung zwischen den Knochenteilen aufgeweiteten Zwischenraum wird das erste Anlageelement 2 in den Brustraum eingeführt. Über die Länge des Zwischenraumes können mehrere derartiger Sternumverschlüsse 1 im Abstand zueinander angelegt werden, so dass über die gesamte Länge des Trennschnittes zwischen den beiden Sternumteilen 19 und 20 eine Fixierung erfolgt.

Nach dem Zusammenspannen der durchtrennten Sternumteile 19 und 20 werden die Haltevorsprünge 15 des ersten Anlageelementes 2 in die Innenflächen der Sternumteile 19 und 20 eingedrückt. Dies geschieht, indem das erste Anlageelement 2 durch Zug an den Raststiften 4 und 5 gegen die Innenseite der Sternumteile 19 und 20 gespannt wird. Durch ein in der Zeichnung nicht dargestelltes Spanninstrument wird anschließend das zweite Anlageelement 3 auf den Raststiften 4 und 5 in Richtung auf das erste Anlageelement 2 verschoben, bis die Haltevorsprünge 14 von der Außenseite der Sternumteile 19 und 20 in diese eingedrungen sind und bis die Oberseite des ersten Anlageelementes 2 und die Unterseite des zweiten Anlageelementes 3 beiderseitig am Sternum anliegen. In diesem angelegten Zustand können die Verlängerungsabschnitte 9 der Raststifte 4 und 5, einschließlich der Brücke 16, entfernt werden. Somit werden die Sternumteile 19 und 20 dauerhaft relativ zueinander fixiert. Eine dauerhafte natürliche Verbindung der beiden fixierten Sternumteile 19 und 20 kann durch knöcherne Verwachsungsprozesse wieder hergestellt werden.

Im Falle einer notwendigen erneuten Trennung des Sternums (Reoperation) werden zunächst die zweiten Anlageelemente 3 entfernt. Danach kann beispielsweise mit einer Knochensäge entlang den Raststiften 4 und 5 eine Durchtrennung des Knochenmaterials des Sternums vorgenommen werden. Durch die Antihaftschicht 17 wird ein Überwachsen des grundsätzlich schwerer zugänglichen ersten Anlageelementes 2 mit Knochen-, Knorpel- und/oder Bindegewebe im Körper des Patienten weitgehend vermieden, wodurch die vollständige Entfernung des Sternumverschlusses 1, einschließlich des ersten Anlageelementes 2, deutlich erleichtert wird. Auf diese Weise kann der reoperative Eingriff ohne größeren Aufwand und insbesondere ohne Komplikationen und ohne Risiken für den betroffenen Patienten durchgeführt werden.

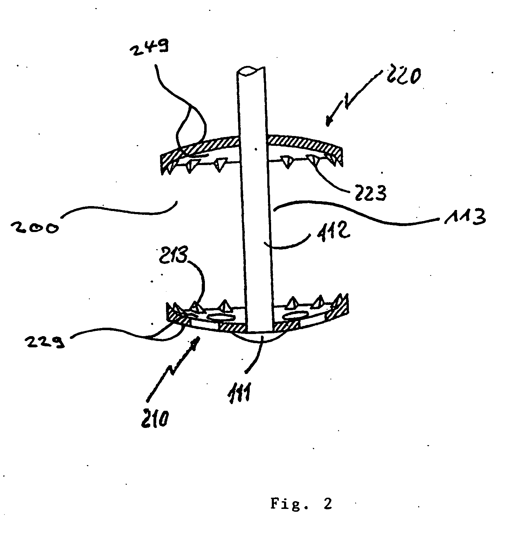

Figur 2 zeigt schematisch ein Implantat 200 zur Fixierung eines aus der Schädelkapsel herausgetrennten Knochenstückes (Kalottensegment) am verbliebenen Schädelbein nach einem operativen Eingriff. Das Implantat 200 weist ein als Stift 113 mit einem Kopf 111 und einem Schaft 112 ausgebildetes Spannelement, ein von innen gegen das verbliebene Schädelbein und das damit wieder zusammenfassende Knochenstück zur Anlage kommende als Scheibe 210 ausgebildetes erstes Anlageelement und ein von außen gegen das Schädelbein und das damit wieder zusammenfassende Knochenstück zur Anlage kommende als Scheibe 220 ausgebildetes zweites Anlageelement auf. Beide Scheiben 210; 220 sind mittig gelocht. Durch die Löcher erstreckt sich im in Figur 2 dargestellten zusammengesetzten Zustand der einzelnen Elemente des Implantats 200 der Schaft 112 des Stiftes 113. Die Ränder der gewölbten Scheiben 210 und 220 weisen Haltevorsprünge in Form von Zacken 213; 223 auf. Die Scheibe 210 ist dabei mit Ausnahme der Zacken 213 vollständig mit einer Antihaftschicht 229 aus einem Polyetherketon beschichtet. Dadurch können Verwachsungen mit dem Dura-Gewebe wirksam unterbunden werden. Die Scheibe 220 ist mit Ausnahme der Zacken 223 vollständig mit einer Beschichtung 249 aus Hydroxylapatit versehen. Durch die Hydroxylapatit-Beschichtung kann in besonders vorteilhafter Weise die Verbindung zwischen dem verbliebenen Schädelbein und dem nach der Operation wieder eingefügten Knochenstück durch Förderung des natürlichen Knochenwachstums unterstützt werden. Zur weiteren Beschleunigung der Verbindung zwischen Schädelbein und eingefügtem Knochenstück bzw. Kalottensegment kann zusätzlich auch der Schaft 112 mit Hydroxylapatit beschichtet sein.

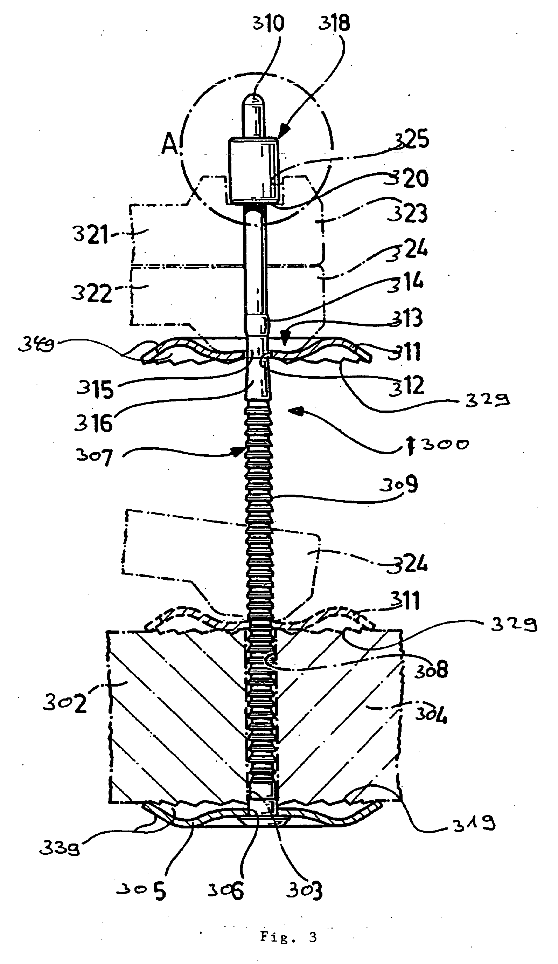

Figur 3 zeigt schematisch ein Implantat 300 zur Einpassung und Fixierung einer Schädelkalotte 302 in eine Öffnung 303 des Schädelknochens 304. Zu diesem Zweck weist das Implantat 300 ein erstes tellerförmiges Anlageelement 305 auf mit einer zentralen Öffnung 306, durch welche ein länglicher, dünner Schaft 307 hindurchgesteckt wird, der relativ zu dem Anlageelement 305 unverschieblich gehalten ist, beispielsweise durch eine Verklebung, eine Verschweißung oder einen Formschluß. Der Außendurchmesser des Schaftes 307 ist so klein, dass der Schaft 307 durch den schmalen Spalt 308 zwischen der Knochenplatte 302 und dem Schädelknochen 304 hindurchpasst. Der Schaft 307 kann als dünner Stab oder kräftiger Draht ausgebildet sein. In dem an das erste Anlageelement 305 anschließenden Bereich weist der Schaft 307 eine größere Anzahl von parallel zueinander verlaufenden Umfangsrippen 309 auf. Außerdem ist vom freien Ende 310 des Schaftes 307 her ein zweites tellerförmiges Anlageelement 311 auf den Schaft 307 aufgeschoben. Dieses zweite Anlageelement 311 weist ebenfalls eine zentrale Öffnung 312 auf, durch die der Schaft 307 hindurchtritt und von dieser Öffnung 312 gehen aus der Zeichnung nicht ersichtliche radiale Einschnitte aus, die angrenzend an die Öffnung 312 lappenförmige Abschnitte in dem tellerförmigen Anlageelement 311 ausbilden, die beim Aufschieben des Anlageelementes 311 entgegen der Aufschieberichtung verbogen werden und mit ihrer Kante am Schaft 307 anliegen. Dadurch kann das Anlageelement 311 nur in eine Richtung auf dem Schaft 307 verschoben werden, und zwar in Richtung auf das erste Anlageelement 305 hin, bei einer Rückbewegung verklemmen sich die Kanten dieser Lappen und verhindern ein Zurückschieben. Dies wird insbesondere dadurch unterstützt, dass die Kanten der Lappen im Bereich der Umfangsrippen 309 in die zwischen den Umfangsrippen 309 ausgebildeten Umfangsnuten eintauchen und somit die Umfangsrippen 309 hintergreifen. Das Anlageelement 305 weist eine Antihaftschicht 339 aus einem Polyetherketon, beispielseweise Polyetheretherketon, auf. Vorzugsweise ist das Anlageelement 305 mit Ausnahme der zackenförmigen Haltevorsprünge 319 mit der Antihaftschicht 339 bedeckt. Auf diese Weise können Gewebeverwachsungen mit dem Dura-Gewebe vermieden werden. Das Anlageelement 311 weist eine Beschichtung 349 aus einem Apatit, insbesondere Hydroxylapatit, auf. Die Apatit-Beschichtung fördert mit besonderem Vorteil die natürliche Knochenverwachsung zwischen der eingefügten Schädelkalotte 302 und dem verbliebenen Schädelknochen 304.

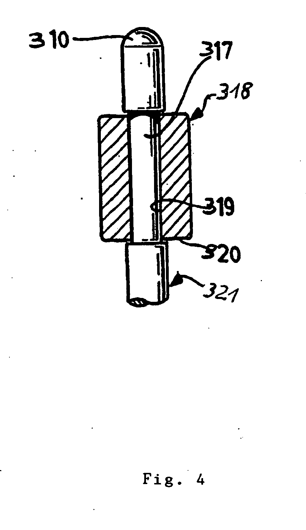

Die im vorherigen Abschnitt genannten Umfangsrippen 309 erstrecken sich über einen Teilbereich des Schaftes 307, der sich unmittelbar an das erste Anlageelement 305 anschließt und etwas mehr als die Hälfte der Schaftlänge abdeckt. Anschließend an den mit Umfangsrippen 309 versehenen Abschnitt ist ein Halteabschnitt 313 vorgesehen, der von oben nach unten eine Umfangsrippe 314, ein Zwischenteil 315 und einen sich konisch erweiternden Übergangsabschnitt 316 aufweist. In diesem Halteabschnitt 313 wird das zweite Anlageelement 311 vor dem eigentlichen Anlegen des Implantats 300 gehalten. Dazu wird das Anlageelement 311 vom freien Ende 310 her auf den Schaft 307 aufgeschoben, bis es über die Umfangsrippe 314 geschoben ist und sich im Zwischenteil 315 befindet. Ein Zurückschieben über die Umfangsrippe 314 ist nicht möglich, ohne größere Verschiebekraft lässt sich das Anlageelement 311 auch nicht über den sich konisch erweiternden Übergangsabschnitt 316 verschieben, d.h. das Anlageelement 311 befindet sich normalerweise im Zwischenteil 315. Zum Anlegen muß eine größere Kraft auf das Anlageelement 311 ausgeübt werden, so dass es über den sich konisch erweiternden Übergangsabschnitt 316 hinweggeschoben wird, wobei dann die Lappen des zweiten Anlageelements 311 in der beschriebenen Weise entgegen der Verschieberichtung verformt werden und beim weiteren Verschiebevorgang über die Umfangsrippen 309 gleiten. Der Schaft 307 ist zwischen dem freien Ende 310 und dem Halteabschnitt 313 zylindrisch ausgebildet und trägt in der Nähe des freien Endes 310 eine Umfangsnut 317 (Figur 4), die als Einstich ausgebildet ist und somit an beiden Seiten stufig endet. Über den Schaft 307 ist ein ringförmiges Anschlagelement 318 geschoben, dessen Höhe der Breite der Umfangsnut 317 entspricht. Das ringförmige Anschlagelement 318 weist eine zentrale Öffnung 319 auf, deren Innendurchmesser im wesentlichen dem Außendurchmesser des Schaftes 307 entspricht, so dass das Anschlagelement 318 ohne weiteres über den Schaft geschoben werden kann. In der Höhe der Umfangsnut 317 wird das ringförmige Element 318 radial nach innen verformt, beispielsweise in eine ovale, linsenförmige oder annähernd quadratische Form (nicht dargestellt), so dass das Anschlagelement 318 zumindest teilweise in die Umfangsnut 317 hineinverformt wird. Eine solche Verformung kann mit einem geeigneten zangenförmigen Werkzeug erfolgen und führt dazu, dass das Anschlagelement 318 durch das Eingreifen in die Umfangsnut 317 in axialer Richtung am Schaft 307 festgelegt wird. Es bildet mit seiner Unterseite 320 einen Vorsprung aus, an dem sich ein Werkzeug eines Anlegeinstrumentes anlegen kann.

Ein solches Anlegeinstrument kann beispielsweise zangenförmig ausgebildet sein und mit zwei gegeneinander verschwenkbaren Branchen 321 und 322, die in Figur 3 lediglich strichpunktiert angedeutet sind. Jeder dieser Branchen 321 und 322 ist an ihrem freien Ende als Anlagekopf 323 bzw. 324 ausgebildet, beide Anlageköpfe sind geeignet zur Anlage des Anlageinstrumentes einmal an dem durch die Unterseite 320 des Anschlagelementes 318 gebildeten Vorsprung und zum anderen an der Oberseite des zweiten Anlegeelementes 311. Werden die beiden Branchen 321 und 322 geöffnet, werden die Anschlagköpfe 323 und 324 voneinander entfernt. Da der Anschlagkopf 323 am Anschlagelement 318 in axialer Richtung fixiert ist, führt dies zu einer Verschiebung des zweiten Anlageelementes 311 bis in die Anlagestellung an der Knochenplatte 302 an dem Schädelknochen 304 (in Figur 3 ist diese Anlageposition gestrichelt dargestellt).

Beide Anlageköpfe 323 und 324 sind im wesentlichen U-förmig ausgebildet, d.h. sie weisen zwei nebeneinander liegende Arme mit einem dazwischenliegenden Spalt auf. Zum Ansetzen des Anlegeinstrumentes wird dieses seitlich so an den Schaft 307 herangeführt, dass dieser in den Spalt zwischen den beiden Armen eintritt. Der Anlagekopf 323 weist weiterhin an seiner Oberseite eine Vertiefung 325 auf, in die bei Anlage des Anlagekopfes 323 am Anschlagelement 318 dieses eintauchen kann, so dass es gegen eine Verschiebung quer zur Längsachse des Schaftes 307 gesichert ist, also insbesondere gegen ein Abgleiten des Anlagekopfes 323 von dem Implantat 300. Bei dem in den Figuren 3 und 4 dargestellten Ausführungsbeispiel wird das Anschlagelement 318 durch ein ringförmiges, separates Bauteil realisiert, das auf den Schaft 307 aufgeschoben und dort axial an ihm festgelegt wird.

## Patentansprüche

1. Implantat (1; 200; 300), insbesondere zur gegenseitigen Fixierung von zwei miteinander zu verbindenden Knochenteilen des Sternums, mit einem ersten Anlageelement (2; 210; 305) zur Anlage an der Innenseite von Knochenteilen, mindestens einem an dem ersten Anlageelement (2; 210; 305) festgelegten, quer von diesem abstehenden Spannelement und einem zweiten Anlageelement (3; 220; 311) zur Anlage an der Außenseite von Knochenteilen, welches mittels des mindestens einen durch einen Zwischenraum zwischen den Knochenteilen hindurchgeführten Spannelements gegen das erste Anlageelement (2; 210; 305) spannbar ist, wobei das Implantat (1; 200; 300) an seiner Oberfläche zumindest teilweise eine Antihaftschicht (17; 229; 339) zur Verhinderung von postoperativen Gewebeadhäsionen aufweist, wobei zumindest das erste Anlageelement (2; 210; 305) einteilig ausgebildet ist, **dadurch gekennzeichnet, dass** die Antihaftschicht (17; 229; 339) ein Polyetherketon aufweist und in Pulverform durch Pulverbeschichtung aufgetragen ist.

2. Implantat (1; 200; 300) nach Anspruch 1, **dadurch gekennzeichnet, dass** die Oberflächen der Anlageelemente (2; 3; 210; 220; 305; 311), insbesondere die Oberfläche des ersten Anlageelements (2; 210; 305), zumindest teilweise die Antihaftschicht (17; 229; 339) aufweisen.

3. Implantat (1; 200; 300) nach Anspruch 1 oder 2, **dadurch gekennzeichnet, dass** an den Anlageelementen (2;3; 210; 220; 305; 311) angeordnete Haltevorsprünge (14; 15; 213; 223; 319; 329) frei von der Antihaftschicht (17; 229; 339) sind.

4. Implantat (1; 200; 300) nach einem der vorhergehenden Ansprüche, **dadurch gekennzeichnet, dass** die Antihaftschicht (17, 229; 339) von einer mindestens einlagigen Antihaftbeschichtung gebildet ist.

5. Implantat (1; 200; 300) nach Anspruch 1, **dadurch gekennzeichnet, dass** es sich bei dem Polyetherketon um ein Polyetheretherketon handelt.

6. Implantat (1; 200; 300) nach einem der vorhergehenden Ansprüche 1 bis 3, **dadurch gekennzeichnet, dass** die Antihaftschicht (17; 229; 339) durch eine Oberflächenbehandlung des Implantats (1; 200; 300) gebildet ist.

7. Implantat (1; 200; 300) nach einem der vorhergehenden Ansprüche, **dadurch gekennzeichnet, dass** das Implantat auf seiner Oberfläche neben der Antihaftschicht 17; 229; 339) zumindest teilweise eine biologisch aktive Beschichtung (249; 349) aufweist.

8. Implantat (1; 200; 300) nach einem der vorhergehenden Ansprüche, **dadurch gekennzeichnet, dass** die Antihaftschicht (17; 229; 339) eine Dicke zwischen 0,1 µm und 0,5 mm besitzt.

9. Implantat (1; 200; 300) nach einem der vorhergehenden Ansprüche, **dadurch gekennzeichnet, dass** die Antihaftschicht (17; 229; 339) antimikrobiell wirksame Substanzen, insbesondere Silber, aufweist.

10. Verwendung eines Gewebeverklebungen verhindernden Materials zur Beschichtung eines Implantats (1; 200; 300) mit einem ersten Anlageelement (2; 210; 305) zur Anlage an der Innenseite von Knochenteilen, mindestens einem an dem ersten Anlageelement (2; 210; 305) festgelegten, quer von diesem abstehenden Spannelement und einem zweiten Anlageelement (3; 220; 311) zur Anlage an der Außenseite von Knochenteilen, welches mittels des mindestens einen durch einen Zwischenraum zwischen den Knochenteilen hindurchgeführten Spannelements gegen das erste Anlageelement (2; 210; 305) spannbar ist, **dadurch gekennzeichnet, dass** es sich bei dem Gewebeverklebungen verhindernden Material um ein Polyetherketon handelt, welches in Pulverform durch Pulverbeschichtung aufgetragen wird.

## Claims

1. Implant (1; 200; 300), particularly for mutually fixing two bone parts of the sternum that are to be connected to each other, with a first bearing element (2; 210; 305) for bearing on the inner face of bone parts, at least one tensioning element secured on the first bearing element (2; 210; 305) and protruding transversely therefrom, and a second bearing element (3; 220; 311) for bearing on the outer face of bone parts, which second bearing element (3; 220; 311) can be tensioned against the first bearing element (2; 210; 305) by means of the at least one tensioning element guided through a space between the bone parts, the implant (1; 200; 300) having, at least on some parts of its surface, an anti-adhesion layer (17; 229; 339) for preventing post-operative tissue adhesions, at least the first bearing element (2; 210; 305) being designed in one piece, **characterized in that** the anti-adhesion layer (17; 229; 339) has a polyether ketone and is applied in powder form by powder coating.

2. Implant (1; 200; 300) according to Claim 1, **characterized in that** the surfaces of the bearing elements (2; 3; 210; 220; 305; 311), in particular the surface of the first bearing element (2; 210; 305), have the anti-adhesion layer (17; 229; 339) at least in some areas.

3. Implant (1; 200; 300) according to Claim 1 or 2, **characterized in that** retaining projections (14; 15; 213; 223; 319; 329) arranged on the bearing elements (2; 3; 210; 220; 305; 311) are free of the anti-adhesion layer (17; 229; 339).

4. Implant (1; 200; 300) according to one of the preceding claims, **characterized in that** the anti-adhesion layer (17; 229; 339) is formed by an at least one-layer anti-adhesion coating.

5. Implant (1; 200; 300) according to Claim 1, **characterized in that** the polyether ketone is a polyether ether ketone.

6. Implant (1; 200; 300) according to one of Claims 1 to 3, **characterized in that** the anti-adhesion layer (17; 229; 339) is formed by a surface treatment of the implant (1; 200; 300).

7. Implant (1; 200; 300) according to one of the preceding claims, **characterized in that**, in addition to the anti-adhesion layer (17; 229; 339), the implant has, on at least some parts of its surface, a biologically active coating (249; 349).

8. Implant (1; 200; 300) according to one of the preceding claims, **characterized in that** the anti-adhesion layer (17; 229; 339) has a thickness of between 0.1 µm and 0.5 mm.

9. Implant (1; 200; 300) according to one of the preceding claims, **characterized in that** the anti-adhesion layer (17; 229; 339) has antimicrobial substances, in particular silver.

10. Use of a material preventing tissue adhesions for coating an implant (1; 200; 300) with a first bearing element (2; 210; 305) for bearing on the inner face of bone parts, at least one tensioning element secured on the first bearing element (2; 210; 305) and protruding transversely therefrom, and a second bearing element (3; 220; 311) for bearing on the outer face of bone parts, which second bearing element (3; 220; 311) can be tensioned against the first bearing element (2; 210; 305) by means of the at least one tensioning element guided through a space between the bone parts, **characterized in that** the material preventing tissue adhesions is a polyether ketone, which is applied in powder form by powder coating.

## Revendications

1. Implant (1 ; 200 ; 300), en particulier pour la fixation réciproque de deux parties d'os du sternum devant être assemblées l'une à l'autre, comportant un premier élément d'appui (2 ; 210 ; 305), destiné à s'appuyer contre la face intérieure des parties d'os, au moins un élément de serrage, fixé au premier élément d'appui (2 ; 210 ; 305), transversalement à distance de ce dernier, et un deuxième élément d'appui (3 ; 220 ; 311), destiné à s'appuyer sur la face extérieure des parties d'os, qui peut être serré, au moyen du ou des éléments de serrage que l'on a fait passer par un espace intermédiaire entre les parties d'os, contre le premier élément d'appui (2 ; 210 ; 305), l'implant (1 ; 200 ; 300) comportant, sur sa surface, au moins partiellement, une couche antiadhésive (17 ; 229 ; 339) destinée à empêcher des adhérences tissulaires postopératoires, au moins le premier élément d'appui (2 ; 210 ; 305) étant réalisé d'une seule pièce, **caractérisé en ce que** la couche antiadhésive (17 ; 229 ; 339) comprend une polyéthercétone et est appliquée sous forme de poudre par revêtement en poudre.

2. Implant (1 ; 200 ; 300) selon la revendication 1, **caractérisé en ce que** les surfaces des éléments d'appui (2 ; 3 ; 210 ; 220 ; 305 ; 311), en particulier la surface du premier élément d'appui (2 ; 210 ; 305), comprennent au moins partiellement la couche antiadhésive (17 ; 229 ; 339).

3. Implant (1 ; 200 ; 300) selon la revendication 1 ou 2, **caractérisé en ce que** des saillies de maintien (14 ; 15 ; 213 ; 223 ; 319 ; 329), disposées contre les éléments d'appui (2 ; 3 ; 210 ; 220 ; 305 ; 311), sont exemptes de la couche antiadhésive (17 ; 229 ; 339).

4. Implant (1 ; 200 ; 300) selon l'une des revendications précédentes, **caractérisé en ce que** la couche antiadhésive (17 ; 229 ; 339) est formée d'un revêtement antiadhésif au moins monocouche.

5. Implant (1 ; 200 ; 300) selon la revendication 1, **caractérisé en ce que**, pour ce qui concerne la polyéthercétone, il s'agit d'une polyétheréthercétone.

6. Implant (1 ; 200 ; 300) selon l'une des revendications précédentes 1 à 3, **caractérisé en ce que** la couche antiadhésive (17 ; 229 ; 339) est formée par un traitement de surface de l'implant (1 ; 200 ; 300).

7. Implant (1 ; 200 ; 300) selon l'une des revendications précédentes, **caractérisé en ce que** l'implant présente sur sa surface, outre la couche antiadhésive (17 ; 229 ; 339), au moins partiellement, un revêtement biologiquement actif (249 ; 349).

8. Implant (1 ; 200 ; 300) selon l'une des revendications précédentes, **caractérisé en ce que** la couche antiadhésive (17 ; 229 ; 339) possède une épaisseur comprise entre 0,1 µm et 0,5 mm.

9. Implant (1 ; 200 ; 300) selon l'une des revendications précédentes, **caractérisé en ce que** la couche antiadhésive (17 ; 229 ; 339) comprend des substances à action antimicrobienne, en particulier de l'argent.

10. Utilisation d'un matériau empêchant les adhérences tissulaires pour revêtir un implant (1 ; 200 ; 300) comportant un premier élément d'appui (2 ; 210 ; 305) destiné à s'appuyer contre la face intérieure de parties d'os, au moins un élément de serrage, fixé au premier élément d'appui (2 ; 210 ; 305), transversalement à distance de ce dernier, et un deuxième élément d'appui (3 ; 220 ; 311), destiné à s'appuyer contre la face extérieure des parties d'os, qui peut être serré, à l'aide de l'au moins un élément de serrage passant par un espace intermédiaire entre les parties d'os, contre le premier élément d'appui (2 ; 210 ; 305), **caractérisée en ce que**, pour ce qui concerne le matériau empêchant les adhérences tissulaires, il s'agit d'une polyéthercétone, qui est appliquée sous forme de poudre par revêtement en poudre.
